(19) **Europäisches Patentamt / European Patent Office / Office européen des brevets**

(11) **EP 2 511 231 B1**

(12) **EUROPEAN PATENT SPECIFICATION**

(45) Date of publication and mention
of the grant of the patent:
**11.03.2015 Bulletin 2015/11**

(21) Application number: **10836238.5**

(22) Date of filing: **10.12.2010**

(51) Int Cl.:
*B82B 1/00* (2006.01)          *B82B 3/00* (2006.01)
*G01N 33/53* (2006.01)        *C12Q 1/68* (2006.01)
*G01N 33/543* (2006.01)

(86) International application number:
**PCT/KR2010/008862**

(87) International publication number:
**WO 2011/071343 (16.06.2011 Gazette 2011/24)**

(54) **HETERODIMER CORE-SHELL NANOPARTICLE IN WHICH RAMAN-ACTIVE MOLECULES ARE LOCATED AT A BINDING PORTION OF A NANOPARTICLE HETERODIMER, USE THEREOF, AND METHOD FOR PREPARING SAME**

KERN-HÜLLE-HETERODIMERNANOPARTIKEL MIT RAMAN-AKTIVEN MOLEKÜLEN AN EINEM BINDUNGSTEIL EINES NANOPARTIKELHETERODIMERS, IHRE VERWENDUNG UND HERSTELLUNGSVERFAHREN DAFÜR

NANOPARTICLE COEUR-ÉCORCE HÉTÉRODIMÈRE DANS LAQUELLE DES MOLÉCULES ACTIVES À EFFET RAMAN SONT SITUÉES AU NIVEAU D'UNE PARTIE DE LIAISON D'UNE NANOPARTICULE HÉTÉRODIMÈRE, UTILISATION DE CELLE-CI, ET PROCÉDÉ DE PRÉPARATION CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.12.2009 KR 20090123017**

(43) Date of publication of application:
**17.10.2012 Bulletin 2012/42**

(73) Proprietors:
• **Korea Research Institute Of Chemical Technology**
**Yuseong-gu, Daejeon 305-343 (KR)**
• **SNU R&DB Foundation**
**Seoul 151-742 (KR)**

(72) Inventors:
• **SUH, Yung Doug**
**Daejeon 305-340 (KR)**
• **NAM, Jwa Min**
**Seoul 151-057 (KR)**
• **LIM, Dong Kwon**
**Seongnam-si**
**Gyeonggi-do 463-400 (KR)**
• **JEON, Ki Seok**
**Daejeon 306-814 (KR)**

(74) Representative: **Kador & Partner**
**Corneliusstraße 15**
**80469 München (DE)**

(56) References cited:
**WO-A1-2009/136741          WO-A2-03/008539**
**WO-A2-2008/097328          JP-A- 2008 524 602**
**KR-A- 20090 116 653**

• **KAHRAMAN ET AL: "Oligonucleotide-Mediated Au-Ag Core-Shell Nanoparticles.", PLASMONICS, vol. 4, 1 January 2009 (2009-01-01), pages 293-301, XP055003757,**
• **YAN CUI ET AL: "Synthesis of Ag c ore Au s hell Bimetallic Nanoparticles for Immunoassay Based on Surface-Enhanced Raman Spectroscopy", THE JOURNAL OF PHYSICAL CHEMISTRY B, vol. 110, no. 9, 1 March 2006 (2006-03-01) , pages 4002-4006, XP055059934, ISSN: 1520-6106, DOI: 10.1021/jp056203x**
• **SANGYEOP LEE ET AL: "Biological Imaging of HEK293 Cells Expressing PLC[gamma]1 Using Surface-Enhanced Raman Microscopy", ANALYTICAL CHEMISTRY, vol. 79, no. 3, 1 February 2007 (2007-02-01), pages 916-922, XP055059933, ISSN: 0003-2700, DOI: 10.1021/ac061246a**

EP 2 511 231 B1

• CAO Y C ET AL: "Nanoparticles with raman spectroscopic fingerprints for DNA and RNA detection", SCIENCE, AMERICAN ASSOCIATION FOR THE ADVANCEMENT OF SCIENCE, WASHINGTON, DC; US, vol. 297, 30 August 2002 (2002-08-30), pages 1536-1540, XP002977805, ISSN: 0036-8075, DOI: 10.1126/SCIENCE. 297.5586.1536
• HUO ET AL: "A perspective on bioconjugated nanoparticles and quantum dots", COLLOIDS AND SURFACES. B, BIOINTERFACES, ELSEVIER, AMSTERDAM, NL, vol. 59, no. 1, 4 July 2007 (2007-07-04), pages 1-10, XP022141891, ISSN: 0927-7765, DOI: 10.1016/J.COLSURFB. 2007.04.019
• G.V.P. KUMAR ET AL: "Hot Spots in Ag Core-Au Shell Nanoparticles Potent for Surface-Enhanced Raman Scattering Studies of Biomolecules", JOURNAL OF PHYSICAL CHEMISTRY C, vol. 111, no. 11, 22 March 2007 (2007-03-22), pages 4388-4392, XP055059937, ISSN: 1932-7447, DOI: 10.1021/jp068253n
• DONG-KWON LIM ET AL: "DNA-embedded Au/Ag core-shell nanoparticles", CHEMICAL COMMUNICATIONS, no. 42, 1 January 2008 (2008-01-01), page 5312, XP055059931, ISSN: 1359-7345, DOI: 10.1039/b810195g
• DONG-KWON LIM ET AL: "Highly uniform and reproducible surface-enhanced Raman scattering from DNA-tailorable nanoparticles with 1-nm interior gap", NATURE NANOTECHNOLOGY, vol. 6, no. 7, 29 May 2011 (2011-05-29), pages 452-460, XP055059781, ISSN: 1748-3387, DOI: 10.1038/nnano.2011.79

**Description**

[Technical Field]

The present invention relates to a core-shell nanoparticle dimer labeled with a Raman active molecule at an interparticle junction. More particularly, the present invention relates to a dimeric nanostructure comprising two nanoparticles, with a Raman active molecule localized at a junction therebetween, each nanoparticle consisting of a gold or silver core with oligonucleotides attached to the surface thereof, and a gold or silver shell sheathing the core. Also, the present invention is concerned with uses of the dimeric nanostructure and a method for preparing the dimeric nanostructure.

[Background Art]

Highly sensitive, accurate detection of single molecules from biological or other samples is being extensively applied to many fields including medical diagnosis, pathology, toxicology, environmental sampling, chemical analysis, etc. Recently, to this end, the biology-chemistry field has widely utilized specifically labeled nanoparticles or chemical materials in studying the metabolism, distribution and binding of small synthetic materials and biomolecules. Typically, radioactive isotopes, organic fluorescent dyes, and quantum dots have been used.

Representative examples of the radioactive isotopes typically useful for research include $^3$H, $^{14}$C, $^{32}$P, $^{35}$S, and $^{125}$I which are respectively used in substitution for $^1$H, $^{12}$C, $^{31}$P, $^{32}$S, and $^{127}$I which are widely distributed in the body. Radioactive isotopes have long been used because radioactive and non-radioactive isotopes have almost the same chemical properties and can be used interchangeably, and because even a small amount of radioactive isotopes can be detected due to their relatively high emission energy. However, radioactive isotopes are difficult to handle because the radiation they produce is harmful to the body. Further, although their emission energy is high, some of the radioactive isotopes have short half-lives so that they cannot be stored for a long period of time or are not suitable for use in long-term experiments.

For an alternative to the radioactive isotopes, an organic fluorescent substance has been used. The organic fluorescent substance absorbs energy of a specific wavelength and emits light at a different characteristic wavelength. Particularly, as detection methods become increasingly simplified, radioactive substances face problems with detection limits and thus require long periods of time for detection. In contrast, an organic fluorescent theoretically allows the detection of even a single molecule because it can emit thousands of photons per molecule under the proper conditions. However, unlike radioactive isotopes, fluorophores cannot substitute for elements of active ligands directly. Instead, they are restrictively designed to be linked to moieties which have no effects on activity in light of the structure activity relationship. Further, fluorescent labels undergo photobleaching with time. Another problem with fluorophores is the interference between different fluorophores because they re-emit a wide spectrum of light wavelengths while being excited over a highly narrow range of wavelengths. Moreover, only a small number of fluorophores are available.

A quantum dot is a semiconductor nanomaterial, which is composed typically of CdSe or CdS as a core and ZnS or ZnSe as a shell, and can emit light of different colors depending on the size of particles and the kind of core materials. Compared to organic fluorescent dyes, quantum dots can be excited with a wider spectrum of excitation wavelength, emit light in a narrower spectrum of wavelengths, and thus show a larger number of different colors. Accordingly, quantum dots have attracted a lot of attention due to their advantages over organic fluorescent dyes. However, quantum dots suffer from the disadvantage of being highly toxic and being difficult to produce on a large scale. In addition, the number of available quantum dots, although theoretically variable, is highly restricted in practice.

To overcome such problems, Raman Spectrometry and/or Surface Plasmon Resonance have been recently used for labeling.

Surface Enhanced Raman Scattering (SERS) is a spectroscopic method which utilizes the phenomenon whereby when molecules are adsorbed on a roughened surface of a metal nanostructure such as a gold or silver nanoparticles, the intensity of Raman scattering is dramatically increased to the level of $10^6$ - $10^8$ times compared with normal Raman signals. As light passes through a transparent medium, molecules or atoms of the medium scatter the light. In this situation, a small fraction of the photons undergo inelastic scattering, known as Raman scattering. For example, a fraction of the incident photons interact with the molecules in such a way that energy is gained or electrons are excited into higher energy levels, so that the scattered photons have a frequency different from that of the incident photons. Because the frequencies of the Raman scattering spectrum account for the chemical compositions and structural properties of the light absorbing molecules in a sample, Raman spectroscopy, together with the nanotechnology which is currently being developed, can be further developed for high sensitive detection of a single molecule. In addition, there is a strong expectation that a SERS sensor can be used importantly as a medical sensor. The SERS effect is in relation with Plasmon resonance. In this context, metal nanoparticles exhibit apparent optical resonance in response to the incident electromagnetic radiation due to the collective coupling of conduction electrons within the metal. Thus, nanoparticles of gold, silver, copper and other specific metals can fundamentally serve as nanoscale antenna for amplifying the localization of electromagnetic radiations. Molecules localized in the vicinity of these particles show far greater sensitivities to Raman spectroscopy.

Accordingly, in addition to highly sensitive DNA analysis, many studies are actively being carried out about using SERS sensors to detect biomarkers including genes and proteins for early diagnosis of various diseases. Raman spectroscopy has various advantages over other methods (Infrared Spectroscopy). Whereas infrared spectroscopy can detect strong signals from molecules which have a dipole moment, Raman spectroscopy allows strong signals to be detected even from non-polar molecules in which induced polarizability is modulated. Hence, almost all organic molecules have their own Raman shifts ($cm^{-1}$). In addition, being free from the interference of water molecules, Raman spectroscopy is suitable for use in the detection of biomolecules including proteins, genes, etc. However, due to low signal intensity, the development of Raman spectroscopy has not yet reached the level where it can be used in practice in spite of research spanning a significant period of time. Since its discovery, Surface-Enhanced Raman Scattering (SERS) has continually been developed to such a level as to detect signals at a molecular level from randomized aggregates of fluorescent dye-absorbed nanoparticles (Science 1997, 275(5303), 1102; Phys rev lett 1997, 78(9), 1667). Since then, many studies of SERS enhancement with various nanostructures (nanoparticles, nanoshells, nanowires) have been reported. In order to utilize SERS as a highly sensitive detection method for a biosensor, Mirkin et al. reported highly sensitive DNA analysis by using DNA-modified gold nanoparticles, with a detection limit of 20 fM (2002, science, 297, 1536). However, there have been almost no advances in preparing single molecule SERS active substrates based on the salt-induced aggregation of silver (Ag) nanoparticles having Raman active molecules (e.g., Rhodamine 6G) since the first study. One report has it that only a fraction (less than 1%) of heterogeneously aggregated colloids has single molecule SERS activity (J Phys Chem B 2002, 106(2), 311). Like this, randomly roughened surfaces provide a multitude of interesting essential data associated with SERS, but this strategy is fundamentally impossible to reproduce because even a small change in the surface morphology leads to a significant change of enhancement. Recently, Fang et al. reported a quantitative measurement of the distribution of site enhancements in SERS. The hottest SERS-active sites ($EF > 10^9$) accounted for only 63 sites out of a total of 1,000,000 sites, but contributed 24% to the overall SERS intensity (Science, 2008, 321, 388). In these regards, assembling SERS-active nanoparticles into well-defined and reproducible hot SERS nanostructures would lead to a highly reliable, sensitive assay for biomolecules and be greatly useful for use in xenodiagnosis and *in vivo* imaging techniques.

However, conventional SERS detection methods for various analytes typically employ colloidal metal particles on substrates and/or supports, for example, aggregated Ag nanoparticles. This arrangement often allows SERS detection at a sensitivity enhanced on the order of $10^6$ - $10^8$, but cannot be applied to single-molecule detection of small analytes, such as nucleotides. In spite of the advantages of SERS, the mechanism behind SERS has not yet been completely understood. Further, SERS-based single-molecule detection generally faces many problems with structural reproducibility and reliability due not only to difficulty in the synthesis and control of well-defined nanostructures, but also to changes of enhancement yield with the wavelength and the polarization direction of the excitation light used for spectrum measurement. Such problems remain as a great hindrance to the application of SERS in the attempt to achieve the development and commercialization of nano-biosensors. In order to solve the above problems, studies for optical properties and precise SERS enhancement controls of well-defined nanostructures are required now more than ever before.

The SERS enhancement studies reported by Jeong, Proke, Schneider, and Lee, et. al., and with a dimer of metal particles, support the theoretical SERS studies on SERS enhancement where SERS results from the very strong electric field (i.e., hot spot or interstitial field) that is formed between at least two nanoparticles. According to a theoretical calculation based on an electromagnetic principle, SERS enhancement of ca. $10^{12}$ times is expected at the hot spot. As such, the enhanced sensitivity of Raman detection, although not apparently homogeneous inside aggregates of colloidal particles, varies depending on the presence of hot spots. However, information about the physical structure of hot spots, the distance range from nanoparticles where enhanced sensitivity is achieved, and sensitivity-enhancing spatial relationship between analytes and aggregated nanoparticles has not been reported anywhere previously. Further, aggregated nanoparticles are unstable in solutions, thus having an opposite effect on the reproducibility of the detection of single-particle analytes.

In addition, even though theoretical simulations and proof-of concept for dimeric structures of gold or silver have been tried, the preparation of a single molecule localized at a junction between nanoparticles has not been reported yet. Synthesis of robust SERS-active nanostructures of gold or silver still remains challenging.

Patent document WO 03/008539 refers to composite core/shell nanoparticles, a two-step method for their preparation and methods of detection of biomolecules comprising the core/shell nanoparticles. In particular, Ag cores (~12 nm in diameter)are disclosed which are coated with one atomic monolayer of Au (~0.3 nm) as shell. The core/shell nanoparticles may further be modified with alkanethiol-oligonucleotides that undergo reversible hybridization with complementary oligonucleotides to form extended nanoparticles network structures including dimers.

Patent document WO 2009/136741 relates to an Au/ Ag core-shell composite including an Au nanoparticle (1 nm-1000 nm), an Ag nanoparticle layer surrounding the Au nanoparticle and a receptor having a target material recognition site bondable or readable with a target material, wherein one end of the receptor is bonded on the surface of the Au nanoparticle, so that a portion of the receptor is embedded into the Ag nanoparticle layer, and the target material recognition site is exposed to the outside of the Ag nanoparticle layer. Further, a method for detecting a target material using the Au/Ag core-shell composite is disclosed. Thereby, the Au nanoparticles can also form dimers via complementary hybridization of oligonucleotides which are bound to the core.

The oligonucleotide-mediated Au-Ag Core-shell nanoparticles described by Kahraman et al. (Plasmonics, 2009, 4, 293) disclose Au core-Ag shell nanoparticles wherein chemically attached Raman reporter molecules and a 12-base-long oligonucleotide are sandwiched between the 13 nm average size Au core and the 9 nm or 21 nm average size Ag shell. That is, both the oligonucleotide and the Raman reporter molecules are found within the Ag shell.

Moreover, Yan Cui et al. (J. Phys. Chem B, 2006, 110, 4002) describe bimetallic Ag core-Au shell nanoparticles which are labeled with monoclonal antibodies and SERS reporter probes for Sandwich Immunoassay analysis.

The publication of Huo et al. (Anal. Chem., 2007, 79, 916) deals with bimetallic Au/Ag core-shell nanoparticles conjugated with monoclonal antibodies and Raman reporter molecules for labeling living cells and highly sensitive SERS imaging of living cells. In particular, the Raman reporter R6G is absorbed on the surface of the Au core and the R6G-modified gold core is then coated with Ag so that the Raman reporter is found within the Ag shell.

Leading to the present invention, intensive and thorough research into the development of nanostructures capable of single-DNA detection with high sensitivity and reproducibility, conducted by the present inventors, resulted in the finding that a dimeric core-shell nanoparticle labeled with a Raman active molecule localized at an interparticle junction, in which the distance between the dimeric nanoparticles is adjusted into a desired range by controlling the thickness of the shell, shows very strong surface-enhanced Raman scattering (SERS) signals, with an SERS enhancement factor (EF) of up to -2.7 x $10^{12}$, and is proven to be a highly reproducible hot-spot particle.

[Disclosure]

[Technical Problem]

It is therefore an object of the present invention to provide a dimeric core-shell nanostructure in which a Raman active molecule is localized at an interparticle junction.

It is another object of the present invention to provide a method for constructing the dimeric nanostructure.

It is a further object of the present invention to provide a method for detecting an analyte using the dimeric nanostructure.

It is still another object of the present invention to provide a kit for detecting an analyte comprising the dimeric nanostructure.

[Technical Solution]

In accordance with an aspect thereof, the present invention pertains to a dimeric core-shell nanostructure labeled with a Raman active molecule localized at an interparticle junction.

In greater detail, the dimeric core-shell nanostructure of the present invention comprises two nanoparticles, each consisting of a core (gold or silver) and a shell (gold or silver) sheathing the core, wherein oligonucleotides are attached to the surface of each nanoparticles and parts of the oligonucleotides are exposed to the outside of the shell, the particles being connected with each other by direct or indirect hybridization between two oligonucleotides. In each nanoparticle, the oligonucleotide is attached at its one terminus to the surface of the core while being partially exposed to the exterior of the shell. The exposed oligonucleotide sequences of the two nanoparticles may be hybridized directly with each other when they are complementary to each other, or indirectly via an oligonucleotide sequence complementary to both the exposed oligonucleotide sequences.

As used herein, the term "core" refers to a metal particle on a surface of which an oligonucleotide is directly attached. Preferably, a gold or silver particle is used. The term "shell" refers to a metal coating layer sheathing the core. A part of the oligonucleotide attached onto the core is within the inside of the shell. Preferably, the shell is made of gold or silver.

Hence, in a preferred embodiment of the present invention, the dimeric core-shell nanostructure is selected from a group consisting of i) a dimeric nanostructure comprising two nanoparticles, each consisting of a gold core and a silver shell, ii) a dimeric nanostructure comprising two nanoparticles, each consisting of a silver core and a gold shell, iii) a dimeric nanostructure comprising two nanoparticles, each consisting of a gold core and a gold shell, and iv) a dimeric nanostructure comprising two nanoparticles, each consisting of a silver core and a silver shell, and v) a dimeric nanostructure comprising two nanoparticles, one consisting of a gold core and a silver shell and the other consisting of a silver core and a gold shell. Most preferably, the dimeric core-shell nanostructure of the present invention comprises two nanoparticles, each consisting of a gold core and a silver shell.

The core-shell nanostructure of the present invention may be in the form of a homodimer or a heterodimer. As used herein, the term "homodimer" refers to a dimeric structure comprising two nanoparticles identical in size and structure to each other, and the term "heterodimer" refers to a dimeric structure comprising two nanoparticles, each different in size or structure from each other.

The diameter of the core particle of the dimeric core-shell nanostructure for Surface Enhanced Raman Scattering nano-labeling in accordance with the present invention is preferably on the order of from 5 nm to 300 nm. When the core has a diameter less than 5 nm, a decreased SERS enhancement effect is obtained. On the other hand, a core diameter exceeding 300 nm would impose many limitations on the biological applications of the nanostructure. More preferably, the core diameter ranges in size from 10 nm to 40 nm. The nanoparticles may be roughly spherical, but may also have an irregular shape or any other kind of shape.

A nano-shell is introduced onto the surface of the core particle. Being adapted to endow the surface of the core particle with an enhanced Raman scattering effect, the nano-shell facilitates Raman spectroscopic analysis. That is, a core particle coated with a nano-shell increases surface enhanced Raman scattering, thus guaranteeing the detection of signals from any chemical materials. Preferably, the shell has a thickness of from 1 nm to 300 nm and more preferably from 1 nm to 20 nm. In addition, the thickness of the shell may increase in proportion with the diameter of the core and the length of the DNA used.

The core is characterized by there being at least one functional oligonucleotide attached to the surface thereof. For example, core A may be functionalized with a protecting oligonucleotide sequence modified with a thiol group at the 3'-terminus and a target-capturing oligonucleotide sequence modified with a thiol group at the 3'-terminus. On the other hand, core B may be functionalized with two different kinds of oligonucleotide sequences (a protecting sequence and a target-capturing sequence, both being modified with a thiol group at the respective 5' termini). In addition, either the target-capturing oligonucleotide attached to core A or core B is modified with a Raman active molecule. Alternatively, oligonucleotides may be attached at a 5'-modified terminus to core A while core B is functionalized with 3'-modified oligonucleotides in accordance with the present invention.

As used herein, the term "protecting oligonucleotide" refers to an oligonucleotide which is attached to the surface of a core particle, stabilizing the core particle with the aim of allowing the target-capturing oligonucleotide to adhere properly to the core surface and to protect it.

As used herein, the term "target-capturing oligonucleotide" refers to an oligonucleotide having a sequence complementary to that of a target oligonucleotide. Both the respective target-capturing oligonucleotides for core A and core B hybridize with a common target oligonucleotide to form a dimeric nanostructure.

The term "target oligonucleotide", as used herein, refers to an oligonucleotide which comprises a sequence complementary to both the target-capturing oligonucleotides for cores A and B, and thus as a linker with which the two target-capturing oligonucleotides hybridize to form a dimeric nanostructure. It should be understood that "target oligonucleotide" does not mean the final target analyte to be analyzed by using the dimeric nanostructure.

Both the protecting oligonucleotide and the target-capturing oligonucleotide may be modified at their 3' or 5' termini with a surface-bound functional group by which they are attached to the surface of the core particle.

As used herein, the term "surface-bound functional group" refers to a compound which is connected to the 3' or 5' terminus of each oligonucleotide and which serves to attach the oligonucletide to the surface of the core particle. As long as it allows the formation of such a small aggregate of nanostructures that the aggregate does not precipitate, any surface-bound functional group may be used without limitations. A surface-bound functional group may be used to cross-link nanostructures as disclosed previously in the art (Feldheim, The Electrochemical Society Interface, Fall, 2001, pp. 22-25). The compound having a surface-bound functional group useful in the present invention comprises at its one end a surface-bound functional group which binds to the surface of the core particle. Preferably, the surface-bound functional group is a sulfur-containing group such as thiol or sulfhydryl (HS). Thus, the functional group may be a compound represented by RSH, an alcohol or phenol derivative in which a sulfur atom is present instead of an oxygen atom. Alternatively, the functional group may be a thiol ester or dithiol ester group respectively represented by RSSR' and RSR' or an amino group ($-NH_2$). In addition, the compound having surface-bound functional group may be linked to a variety of reactive groups, e.g., $-NH_2$, -COOH, -CHO, -NCO, and an epoxide group, which can react with biomolecules such as DNA probes, antibodies, oligonucleosides and amino acids. These reactive groups are well known in the art and may be applied to the method and apparatus of the present invention.

On the other hand, the oligonucleotide may contain a spacer sequence at the end opposite to the linker compound. The spacer sequence not only prevents the core-coating shell from covering the target recognition sequence of the target-capturing oligonucleotide, but also provides a space for a proper shell thickness. An example of the spacer sequence is A10-PEG.

As used herein, the term "Raman active molecule" refers to a molecule which facilitates the detection and measurement of an analyte by a Raman detector when the dimeric nanostructure of the present invention is applied to one or more analytes. The target-capturing oligonucleotide on either core A or core B is modified with a Raman active molecule. The Raman active molecule produces a specific Raman spectrum and has the advantage of allowing the effective analysis of subsequent biomolecules.

As Raman active molecules useful in Raman spectroscopy, organic or inorganic molecules, atoms, complexes or synthetic molecules, dyes, natural dyes (phycoerythrin, etc.), organic nanostructures such as $C_{60}$, buckyballs, carbone nanotubes, quantum dots, and organic fluorescent molecules may be used. Specific examples of the Raman active molecules include FAM, Dabcyl, TRITC (tetramethyl rhodamine-5-isothiocyanate), MGITC (malachite green isothiocyanate), XRITC (X-rhodamine-5-isothiocyanate), DTDC (3,3-diethylthiadicarbocyanine iodide), TRIT (tetramethyl rhodamine isothiol), NBD (7-nitrobenz -2-1,3-diazole), phthalic acid, terephthalic acid, isophthalic acid, para-aminobenzoic acid, erythrosine, biotin, digoxigenin, 5-carboxy-4',5'-dichloro-2',7'-dimethoxy, fluorescein, 5-carboxy-2',4',5',7'-tetrachlorofluorescein, 5-carboxyfluorescein, 5-carboxyrhodamine, 6-carboxyrhodamine, 6-carboxytetramethyl aminophthalocyanine, azomethine, cyanine (Cy3, Cy3.5, Cy5), xanthine, succinylfluorescein, aminoacridine, quantum dots, carbon allotropes, cyanides, thiol, chlorine, bromine, methyl, phosphor and sulfur, but are not limited thereto. For use in the dimeric nanostructure of the present invention, a Raman active molecule is required to show a clear Raman spectrum and must be associated or related with different kinds of analytes. Prefered are cyanine type fluorescent dyes such as Cy3, Cy3.5 and Cy5, or organic fluorescent molecules such as FAM, Dabcyl, rhodamine molecules, etc. These organic fluorescent molecules have the advantage of detecting higher Raman signals by being resonant with excitation laser wavelengths used for Raman analysis. Raman active molecules may be attached to an analyte directly or via a linker compound.

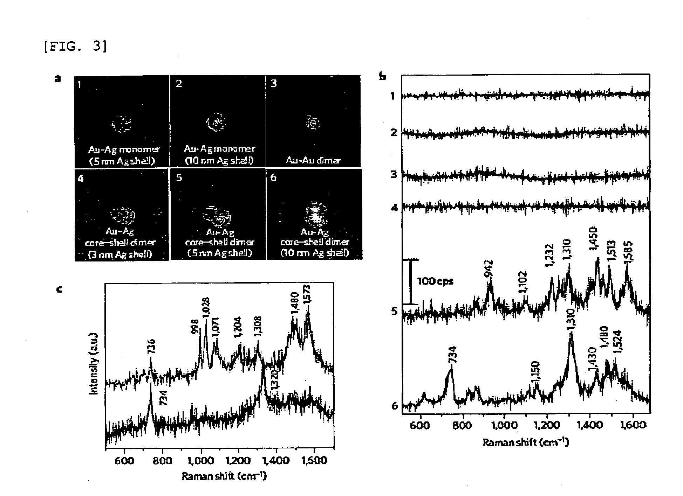

In accordance with an aspect of the present invention, it was found that only when a Raman active molecule is localized at an interparticle junction can the nanostructure detect SERS signals. For example, no SERS signals could be detected for core-shell monomers because they have no hot spots and only one Raman active molecule was present (FIGS. 3A-1, 2).

In accordance with another aspect thereof, the present invention pertains to a method for constructing a dimeric core-shell nanostructure labeled with a Raman active molecule.

In greater detail, the method for constructing a dimeric core-shell nanostructure labeled with a Raman active molecule comprises: 1) synthesizing core A and core B, respectively, core A having a protecting oligonucleotide and a target-capturing oligonucleotide which are bound to a surface thereof, core B having a protecting oligonucleotide and a target-capturing oligonucleotide modified at one terminus with a Raman active molecule which is bound to a surface thereof, wherein the protecting oligonucleotide is an oligonucleotide which is attached to the surface of a core particle, stabilizing the core particle with the aim of allowing the target-capturing oligonucleotide to adhere properly to the core surface and to protect it and wherein the target-capturing oligonucleotide is an oligonucleotide having a sequence complementary to that of a target oligonucleotide, 2) hybridizing core A and core B with a target oligonucleotide to form a dimeric structure, and 3) introducing a shell on each of core A and core B.

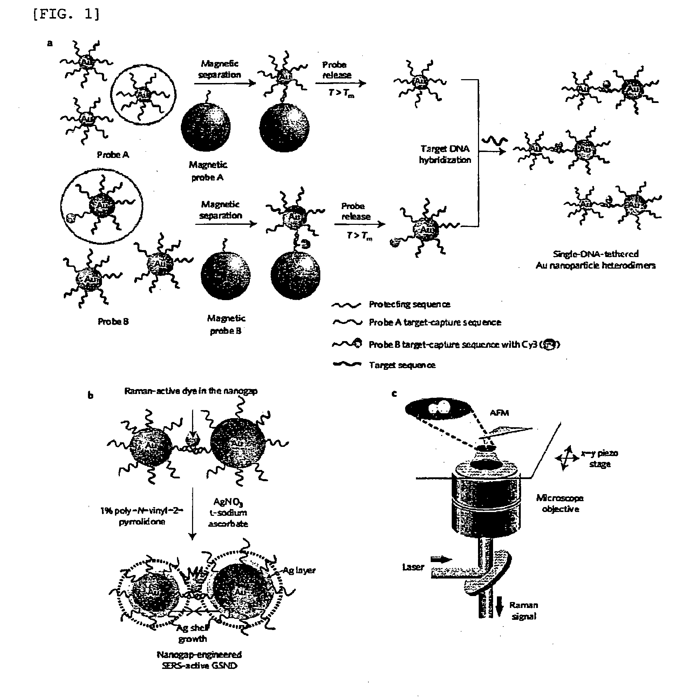

In the first step, core A and core B, each having a protecting oligonucleotide and a target-capturing oligonucleotide bound to a surface thereof, are synthesized. In the synthesis of the core particles according to an embodiment of the present invention, gold core A is functionalized with two kinds of DNA sequences modified at the 3' termini with a thiol group (a protecting oligonucleotide sequence and a target-capturing oligonucleotide sequence). Likewise, gold core B is functionalized with two kinds of DNA sequences modified at the 5' termini with a thiol group. The molar ratios of the two kinds of sequences (protecting sequence/target-capture sequence) were 99:1 for core A and 199:1 for core B. These ratios were adopted to modify one target-capturing oligonucleotide per probe on the basis of nanoparticle size-dependent DNA loading capacity (FIG. 1A). Importantly, the Raman active Cy3, FAM or Dabcy1 dye was preconjugated to the target-capturing oligonucleotide bound to core B.

In order to remove the nanoparticle monomer to which no target capturing sequences are bound, the oligonucleotide-modified cores can be purified by a magnetic-separation process. Tosyl-modified magnetic beads (diameter 1 $\mu$m, Invitrogen) can be functionalized by amine-modified target oligonucleotide sequences complementary to the target-capturing sequences for cores A and B, respectively. Only the core particles having target-capturing sequences bound thereto form complexes with the magnetic beads by hybridization. After the hybridization reaction, an external magnetic field is applied to the reaction solution to separate the complexes of cores and magnetic beads. The cores are released from the magnetic beads by heating the complexes to a temperature higher than the melting point (Tm) of the hybridized double-stranded DNA sequences.

In the second step, the cores A and B are allowed to form a dimer by hybridization with a target oligonucleotide sequence. In the first step, the core particles A and B separated by and isolated from magnetic beads in a buffer, e.g. 0.3 M PBS, are hybridized with a sufficient amount of a target oligonucleotide sequence to form a desired dimeric nanostructure. Thus, the method of the present invention can produce the dimer at high yield (70-80%).

In the third step, the introduction of a nano-shell on the core particles may be preferably conducted by reacting a gold core particle precursor with a silver nanoparticle precursor at 10-100°C in a solvent. Preferably, the silver nanoparticle precursor is selected from among $AgNO_3$ and $AgClO_4$. As long as it contains Au ions, any compound may be used as a precursor of the gold core particles. Preferable is $HAuCl_4$. Silver ions or gold ions can be converted into gold or silver nanoparticles by a reducing agent. Examples of the reducing agent useful in the present invention include

hydroquinone, sodium borohydride (NaBH$_4$), and sodium ascorbate, but are not limited thereto. A solvent suitable for use in the formation of the nano-shell is preferably an aqueous solution (pure water or phosphate buffer). Additionally, a stabilizer may be used to precisely control the thickness of the nano-shell. A reaction temperature less than 10°C takes too much time for the formation of silver nanoparticles. On the other hand, when the reaction temperature exceeds 100°C, irregular silver nanoparticles are formed. Thus, the precursors are preferably reacted within the given temperature range. The reaction may be conducted for 10 to 24 hrs depending on the reaction temperature.

The dimeric core-shell nanostructure labeled with a Raman active molecule at an interparticle junction in accordance with the present invention is functionalized at a surface thereof or a surface of the core with a probe molecule capable of recognizing an analyte, so that it can be applied to the detection of various biomolecules.

Examples of the analytes are amino acids, peptides, polypeptides, proteins, glycoproteins, lipoproteins, nucleosides, nucleotides, oligonucleotides, nucleic acids, saccharides, carbohydrates, oligosaccharides, polysaccharides, fatty acids, lipids, hormones, metabolites, cytokines, chemokines, receptors, neurotransmitters, antigens, allergens, antibodies, substrates, co-factors, inhibitors, drugs, pharmaceutical substances, nutrients, prions, toxins, toxic substances, explosive substances, pesticides, chemical weapon agents, biologically noxious agents, radioactive isotopes, vitamins, heterocyclic aromatic compounds, oncogenic agents, mutagenic factors, anesthetics, amphetamine, barbiturate, hallucinogens, wastes, and contaminants. When the analytes are nucleic acids, they include genes, viral RNAs and DNAs, bacterial DNAs, fungal DNAs, mammal DNAs, cDNAs, mRNAs, RNA and DNA fragments, oligonucleotides, synthetic oligonucleotides, modified oligonucleotides, single- and double-stranded nucleic acids, and natural or synthetic nucleic acids.

Non-limiting examples of the analyte-recognizing probe molecules bound to the surface of the dimeric nanostructure include antibodies, antibody fragments, genetically engineered antibodies, single-chain antibodies, receptor proteins, ligand proteins, enzymes, inhibitor proteins, lectins, cell-adhesion proteins, oligonucleotides, polynucleotides, nucleic acids, and aptamers.

The entire dimeric nanostructure of the present invention may be coated with an inorganic material. After being entirely coated with an inorganic material, the dimeric nanostructure of the present invention can withstand greater structural deformation factors. Hence, the entire inorganic coating stabilizes the dimeric nanostructure and is beneficial for the storage and use of the dimeric nanostructure. As long as it has no influence on Raman signals, any inorganic material may be used. Preferable as the inorganic material is silica.

In accordance with a further aspect thereof, the present invention provides a use of the dimeric nanostructure according to the present invention for detecting an analyte.

In greater detail, the use comprises 1) functionalizing a surface of the dimeric nanostructure or the core of the dimeric nanostructure with a probe molecule capable of detecting an analyte; 2) exposing the dimeric nanostructure to a sample containing at least one analyte; and 3) detecting and identifying the analyte by laser excitation and Raman spectroscopy.

Preferably, the analytes are detected and identified using any well-known Raman spectroscopy. Examples of the Raman spectroscopy useful in the present invention include SERS (Surface Enhanced Raman Scattering), SERRS (Surface Enhanced Resonance Raman Spectroscopy), hyper-Raman and/or CARS (Coherent Anti-Stokes Raman Spectroscopy).

The term "Surface Enhanced Raman Scattering" (SERS) refers to a spectroscopic method which utilizes a phenomenon whereby when molecules are adsorbed on a roughened surface of a metal nanostructure such as gold or silver nanoparticles or are present within a distance of hundreds of nanometers from a surface, the intensity of Raman scattering is dramatically increased to the level of $10^6 - 10^8$ times compared with normal Raman signals. The term "Surface Enhanced Resonance Raman Spectroscopy" (SERRS) refers to a combination of SERS and resonance Raman spectroscopy that uses proximity to a surface to increase Raman intensity, and an excitation wavelength matched to the maximum absorbance of the molecule being analyzed. The term "Coherent Anti-Stokes Raman Spectroscopy" (CARS) refers to a spectroscopic method in which two laser beams, variable and fixed, are incident on a Raman active medium to generate a coherent anti-Stokes frequency beam.

In an embodiment, the detection method of analytes in accordance with the present invention comprises 1) synthesizing the dimeric nanostructure of the present invention; 2) functionalizing a surface of the dimeric nanostructure or the core with a probe molecule complementary to a nucleic acid analyte to be detected; 3) isolating, purifying and amplifying the nucleic acid analyte from a sample; 4) hybridizing the dimeric core-shell nanostructure with a specific sequence of the amplified nucleic acids; and 5) detecting and identifying the nucleic acid analyte combined with the dimeric nanostructure using Raman spectroscopy. When being modified suitably for analyte conditions, the method may be applied to the detection of at least one single-nucleotide polymorphism (SNP) or other genetic mutations from a sample and further applied to DNA sequencing.

In an embodiment used in practice, the Raman active substrate may be operably linked with at least one Raman detection unit device. Raman spectroscopy-based methods detecting analytes are well known in the art (e.g., U. S. Patent Nos. 6,002,471, 6,040,191, 6,149,868, 6,174,677, and 6,313,914). In SERS and SERRS, the intensity of Raman scattering from molecules absorbed on a roughened metal surface such as silver, gold, platinum, copper or aluminum is increased by $10^6$ fold or higher compared with normal Raman signals.

Non-limiting examples of the Raman detection apparatus are disclosed in U. S. Patent No. 6,002,471. The excitation light is generated by either a Nd:YAG laser at 532 nm wavelength or a Ti:sapphire laser at 365 nm wavelength. Pulsed laser beams as well as continuous beams can be used. The light excitation signal passes through confocal optics 6 and the microscope objective, and is focused onto a Raman active substrate containing at least one analyte. The Raman light emitted from the analyte is collected by the microscope objective and the confocal optics, and is coupled to a monochromator for spectral dissociation. The confocal optics includes a combination of dichroic filters, barrier filters, confocal pinholes, objective lenses, and mirrors, and serves the purpose of reducing the background signal. Standard full field optics as well as confocal optics can be used. The Raman emission signals are detected by a detector system which includes an avalanche photodiode interfaced with a computer for counting and the digitization of the signals.

Another example of the detection apparatus may be found in U.S. Patent No. 5,306,403 in which the SERS measurements can be conducted with a Spex Model 1403 double-grating spectrometer equipped with a gallium-arsenide photomultiplier tube (RCA, Model C31034 or Burle Industries Model C3103402) which is operated in single-photon counting mode. The laser source is a 514.5 nm line argon-ion laser (SpectraPhysics, Model 166) and a 647.1 nm line of a krypton-ion laser (Innova 70,Coherent).

Other lasers available for excitation include the nitrogen laser (Laser Science Inc.) at 337 nm, and the helium-cadmium laser (Liconox) at 325 nm (U. S. Patent No. 6,174,677), photodiodes, Nd:YLF laser, and/or various ion lasers and/or dye lasers. The beams are spectrally purified with a bandpass filter (Corion) and collimated before being focused onto a Raman active substrate with a 6X objective lens (Newport, Model L6X). Furthermore, the objective lens is used both to excite the analyte and to collect the Raman signals. This end-on excitation/collection geometry was made possible by using a holographic beam splitter (Kaiser Optical Systems, Inc., Model HB 647-26N18). A holographic notch filter (Kaiser Optical Systems, Inc., HNPF-647-1.0) can be placed in the SERS signal beam to further reject Rayleigh scattered radiation. Another Raman detector is a spectrograph (ISA, HR-320) equipped with a red-enhanced intensified charge-coupled device (RE-ICCD) detection system (Princeton Instruments). Other detectors such as a Fourier transform spectrometer (based on Michelson interferometer), a charged injection device (CID), photodiode arrays, InCaAs detectors, electron-multiplying CCD, highly sensitive CCD and/or phototransistor arrays may be used.

Any well-known suitable form or modification of Raman spectroscopy or related spectrometry may be used for the detection of analytes. Examples of the Raman spectroscopy include normal Raman scattering, resonance Raman scattering, surface enhanced Raman scattering, surface enhanced resonance Raman scattering, coherent anti-Stokes Raman spectroscopy, stimulated Raman spectroscopy, inverse Raman spectroscopy, stimulated gain Raman spectroscopy, hyper-Raman scattering, molecular optical laser examiner (MOLE), Raman microprobing, Raman microscopy, confocal Raman microspectrometer, 3-D or scanning Raman, Raman saturation spectroscopy, time resolution resonance Raman, Raman dissociation spectroscopy, and UV-Raman microscopy, but are not limited thereto.

According to an embodiment of the present invention, the Raman detection apparatus may comprise a computer. No limitations are imparted to the computer used in the present invention. An illustrative computer may comprise a bus for interchanging information and a processor for processing information. The computer may further comprise RAM or another dynamic storage device, ROM or another static storage device, and a data storage device such as a magnetic disc or an optical disc, together with a corresponding driver. Also, the computer may comprise peripheral devices such as a display (e.g., a cathode ray tube or a liquid crystal display), an alphabet input device (e.g., keyboard), a cursor pointing device (e.g., mouse, trackball, or cursor key), and a communication device (e.g., modem, network interface card or Ethernet, token ring or other devices interfaced with a network).

In an embodiment of the present invention, the Raman detection apparatus may be operably linked to a computer. Data from the detection apparatus may be processed by the processor and stored in the main memory device. Data on the emission profiles for standard analytes may be stored on the main memory device or ROM. The processor can identify the analyte from the sample by comparing emission spectra from the analyte in the Raman active substrate. The processor can analyze the data from the detection apparatus to identify and quantify various analytes. Differently set computers may be used to serve different purposes. Hence, the structure of the Raman spectroscopy system may differ from one embodiment to another. After being collected, data are typically transferred to a device where data are analyzed. For data analysis, the data from the detector are processed by a digital computer as described above. Typically, the computer is programmed so as to receive and store the data from the detector as well as analyze and process the data.

In accordance with still a further aspect thereof, the present invention pertains to a kit for detecting an analyte, comprising the dimeric nanostructure of the present invention.

For example, when the analyte to be detected is a nucleic acid, the kit may comprise ingredients necessary for RT-PCR to amplify the nucleic acid contained in a sample. The RT-PCR kit may further comprise a pair of primers specific for the nucleic acid analyte, a test tube or another proper container, a reaction buffer (various pH values and Mg concentrations), deoxynucleotides (dNTPs), enzymes such as Taq-polymerase and reverse transcriptase, DNase and RNase inhibitors, DEPC-water, and sterilized water. In a preferred embodiment of the present invention, the detection kit may be designed to conduct a DNA chip function. The DNA chip kit may comprise a substrate on which genes or cDNAs corresponding to fragments of the genes are arranged, and reagents, formulations and enzymes for constructing fluorescent probes. Also, the substrate may further comprise a control gene or a cDNA corresponding to a fragment of the gene.

In another embodiment of the present invention, when the analyte to be detected is a protein, the kit may be designed for the immunological detection of an antibody and may comprise a substrate, a proper buffered solution, a secondary antibody conjugated with the dimeric nanostructure of the present invention, and a coloring agent. The substrate may be treated on a nitrocellulose membrane, a 96-well plate made of polyvinyl resin, a 96-well plate made of polystyrene resin, or a slide glass.

As a matter of course, the detection kit comprises general tools and agents well known in the art. Examples of the tools/agents include a carrier, a labeling substance capable of producing a detectable signal, a dissolving agent, a washing agent, a buffered solution, and a stabilizer, but are not limited thereto. In the case where the labeling substance is an enzyme, a substrate for measuring the activity of the enzyme and a reaction terminator may be contained in the kit. Examples of the carrier include, but are not limited to, soluble carriers, for example, a well-known, physiologically acceptable buffer, e.g., PBS, insoluble carriers, for example, polystyrene, polyethylene, polypropylene, polyester, polyacrylonitrile, fluorine resin, crosslinked dextran, polysaccharides, polymers such as magnetic beads in which latex is coated with metal, paper, glass, agarose or a combination thereof.

For the formation of antigen-antibody complexes, well-known methods may be employed. Examples of the methods include histochemical staining, RIA, ELISA, Western blotting, immunoprecipitation assay, immunodiffusion assay, complement fixation assay, FACS, and protein chip, but are not limited thereto.

[Advantageous Effects]

Because a Raman active molecule is localized at an interparticle junction and the distance between the Raman active molecule and the nano-core particle is precisely adjusted by the thickness of the silver or gold shell, the dimeric core-shell nanostructure of the present invention allows the production of strong surface enhanced Raman scattering (SERS) signals. Further, in spite of the localization of only one Raman active molecule, strong Raman signals can be detected using the dimeric core-shell nanostructure. In addition, the method for constructing the dimeric core-shell nanostructure guarantees the production of the dimer with high purity. Particularly, the nanostructure can be constructed at high purity by the stoichiometric control of oligonucleotides for cores A and B, and by the use of magnetic nanobeads when purifying the cores A and B. Further, the gap between the two nanoparticles can be adjusted at the nano level. Being designed to amplify Raman signals by a large degree, the core-shell nanostructure of the present invention finds application in various fields, including the detection of analytes such as DNA and proteins (biomarkers) associated with the onset and progression of specific diseases, large-scale genome sequence analysis, single-nucleotide polymorphism (SNP) detection, base sequencing, gene fingerprinting, disease relationship, and drug development.

[Description of Drawings]

FIGS. 1A and 1B are schematic diagrams showing the synthesis of Au nanoparticle dimers through magnetic purification, DNA hybridization and Ag-shell formation. The protecting sequence for probe A is 3'-HS-$(CH_2)_3$-$\underline{A_{10}}$-$\underline{PEG_{18}}$-AAACTCTTTGCGCAC-5', the target-capturing sequence for probe A is 3'-HS-$(CH_2)_3$-$\underline{A_{10}}$-$\underline{PEG_{18}}$-CTCCCTAATAACAAT-5', and the modified sequence for MMP-A is 3'-$NH_2$-$(CH_2)_3$-$\underline{A_{10}}$-$\underline{PEG_{18}}$-ATTGTTATTAGGGAG-5'(Tm=38°C). The protecting sequence for probe B is 5'-HS-(CH2)6-$\underline{A_{10}}$-$\underline{PEG_{18}}$-AAACTCTTTGCGCAC-3', the target-capturing sequence for probe B is 5'-HS-$(CH_2)_3$-$\underline{A_{10}}$-$\underline{PEG_{18}}$-ATCCTTATCAATATTAAA-Cy3-3' and the modified sequence for MMP-B is 5'-$NH_2$-$(CH_2)_3$-$\underline{A_{10}}$-$\underline{PEG_{18}}$-TTTAATATTGATAAGGAT-3' (Tm=40°C) . The underlined parts represent spacer sequences designed to facilitate Ag-shell formation. The target-DNA sequence is 5'-GAGGGATTATTGTTAAATATTGATAAGGAT-3' (anthrax oligonucleotide). FIG. 1C shows an AFM-correlated nano-Raman spectroscopy setup (laser focal diameter 250 nm) for the identification of SERS hot-spot from a single dimeric nanostructure.

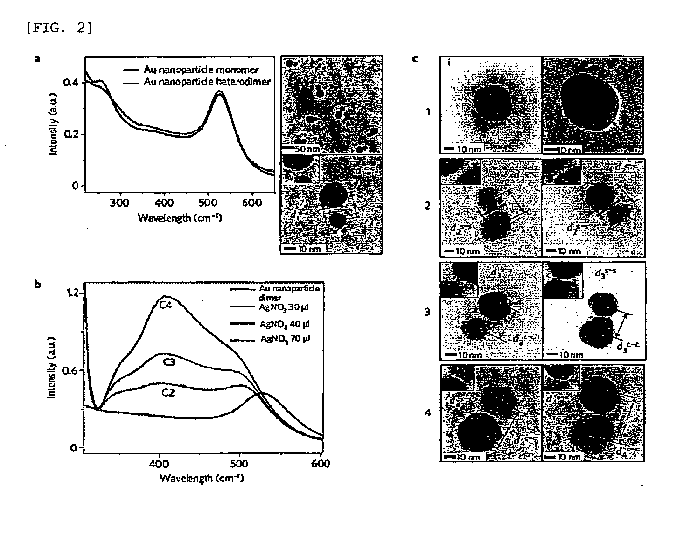

FIG. 2A shows UV-visible spectra before and after Au nanoparticle dimer formation and the corresponding TEM and HR-TEM images. FIG. 2B shows UV-visible spectra before and after the introduction of an Ag-shell on the Au nanoparticle dimer and the corresponding TEM and HR-TEM images. FIG. 2C shows Plasmon resonance (peak at ~400 nm) of the nanostructure which varies depending on the silver-shell thickness. They correspond to HR-TEM images of Au-Ag core-shell dimer. The HR-TEM images C.1a and C.1b of the core-shell nanostructure represent Au-Ag core-shell monomers with a shell thickness of 5 nm and 10 nm, respectively. C.2, C.3 and C.4 are respectively Au-Ag core-shell heterodimers with a shell thickness of ~3 nm, ~5 nm, and ~10 nm.

FIG. 3A shows an AFM (atomic force micrograph, 1 x 1 $\mu$m) of the Au-Ag core-shell monomer and the heterodimer. FIG. 3B shows correlated SERS spectra taken from the monomeric or dimeric Au-Ag core-shell nanostructures. FIG. 3C shows all spectra taken with a 514.5 nm excitation laser, 1 s accumulation, 100 $\mu$W sample, and a 250 nm laser focal diameter. Raman spectra were taken from Cy3-modified oligonucleotides (red line) and Cy3-free oligonucleotides (black line) in NaCl-aggregated silver colloids.

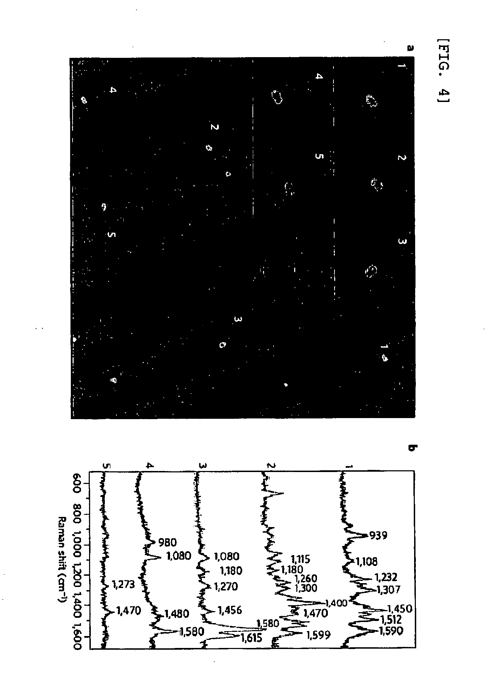

FIG. 4 shows SERS signals from individual, analyzed Au-Ag core-shell dimers which correspond to single-molecule active Cy3. FIG. 4A shows the tapping-mode AFM images (5 $\mu$m X 5 $\mu$m) of the Au-Ag core-shell dimer (corresponding to the nanostructure with an Ag-shell thickness of ~ 5nm and a gap of ~1 nm in FIG. 2B-2). FIG. 4B shows SERS spectra of Cy3 from the individual dimeric nanostructure with a laser wavelength of 514.5 nm, a laser power of ~80 $\mu$W, a laser focal diameter of ~250 nm, and an integration time of 1 s.

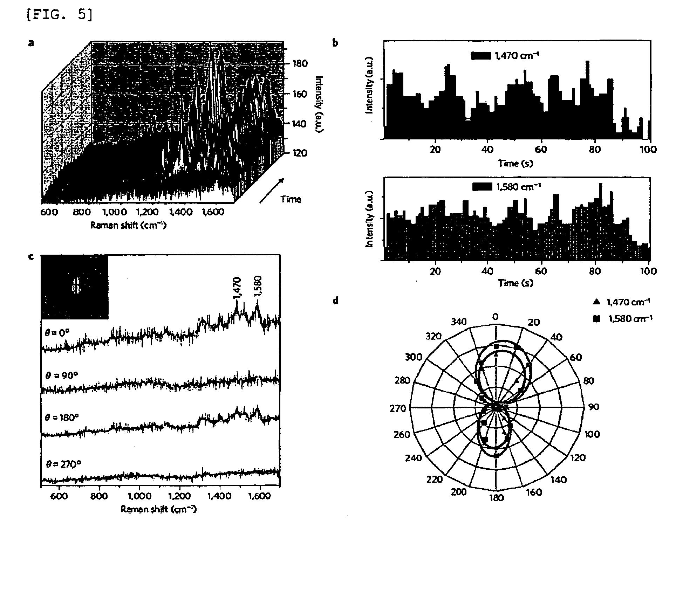

FIGS. 5A and 5B show blinking SERS spectra taken from the nanostructure with an accumulation time of 1 s. FIG. 5C shows SERS spectra taken from Au-Ag core-shell heterodimers with different incident-laser polarizations. FIG. 5D shows polar plots of integrated SERS intensities of the 1470 and 1580 cm$^{-1}$ Raman bands with respect to $\theta$. They were measured with a laser wavelength of 514.5 nm, a laser power of -40 $\mu$W, a laser focal diameter of ~250nm, and an integration time of 20 s.

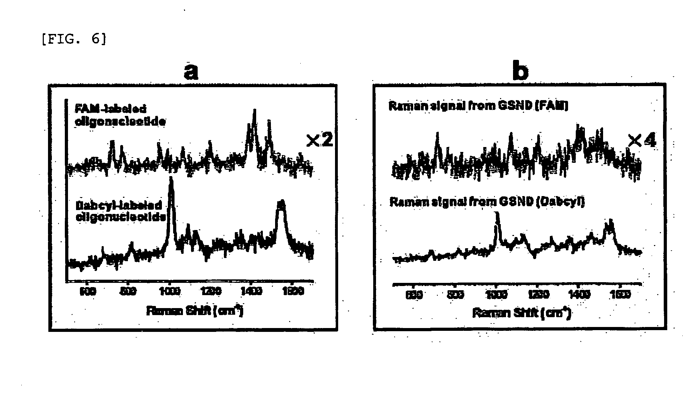

FIG. 6 shows SERS spectra from dimeric nanostructures modified with oligonucleotide labeled with FAM and Dabcyl. FIG. 6A shows Raman spectra from FAM-labeled oligonucleotides (1 nM) and Dabcy1-labeled oligonucleotides (1 nM) in solutions. FIG. 6B shows Raman spectra from dimeric Au-Ag core-shell nanostructures labeled with FAM (Ag-shell 5 nm) and Dabcyl (Ag shell, 5 nm).

A better understanding of the present invention may be obtained through the following examples which are set forth to illustrate, but are not to be construed as limiting the present invention.

[Mode for Invention]

The above and other objects, features and other advantages of the present invention will be more clearly understood from the following examples, but it should be understood that the present invention is not limited to the following examples in any manner.

## EXAMPLE 1: Preparation of Dimeric Au-Ag Core-Shell Nanostructure Labeled with Raman Active Molecule (Cy3) localized at Interpacticle Junction

Based on a DNA-directed bridging method, the synthesis of a Raman active Au-Ag core-shell dimer was conducted using target oligonucleotide-tethered Au nanoparticles, with an Ag-shell being formed from a controlled amount of Ag precursor (FIG. 1).

Firstly, highly purified Au nanoparticle heterodimers were produced by precisely controlling the molar ratio between the protecting oligonucleotide and the target-capturing oligonucleotide, followed by an effective purification. Because the maximum distance (gap distance) between the Au nanoparticle (AuNP) surface and the Cy3 molecule still remained 7.5 nm, it needed to be decreased so as to give an amplified electromagnetic enhancement. A silver nanoshell was introduced since silver enhances SERS signals several times more than gold.

In detail, the DNA tethered Au nanoparticle dimers were coated with silver by means of a well-known nanometer-scale silver-shell deposition process (Chem. Comm. 2008, J. Phys. Chem. B 2004, 108, 5882-5888) on the Au nanoparticle surface to shorten the distance between the nanoparticles, which leads to the amplification of SERS signals. In this regard, a 250 $\mu$M Au nanoparticle dimer solution was reacted with various amounts of AgNO$_3$ solution [10$^{-3}$ M] at room temperature for 3 h in the presence of 100 $\mu$L of polyvinyl pyrrolidone as a stabilizer and 50 $\mu$L of L-sodium ascorbate [10$^{-1}$ M] as a reductant in a 0.3 M PBS solution. The Ag shell thicknesses of the Au-Ag core-shell nanoparticles were ~3 nm, ~5 nm and ~10 nm when using 30 $\mu$L, 40 $\mu$L, and 70 $\mu$L of an AgNO$_3$ solution [10-3 M], respectively. In this manner, target oligonucleotide-tethered Au-Ag core-shell heterodimeric nanostructures with an Ag shell thickness of ~3 nm, - 5 nm and ~ 10 nm were synthesized.

A gold nanoparticle (15 nm) for probe A was functionalized with two kinds of 3'-thiol-modified oligonucleotides in such a manner that one target-capturing sequence was assigned to the surface of the gold nanoparticle. Also, a gold nanoparticle (30 nm) for probe B was functionalized with two kinds of 5'-thiol-modified oligonucleotides. The molar ratios of the two kinds of sequence (protecting sequence/target-capture sequence) were 99:1 for core A and 199:1 for core B. These ratios were adopted to modify one target-capturing oligonucleotide per probe on the basis of nanoparticle size-dependent DNA loading capacity (FIG. 1). Importantly, the target-capturing sequence for probe B was labeled at the terminus with Cy3 which serves as a Raman tag. In order to remove the nanoparticle monomer to which no target capturing sequences are bonded, the oligonucleotide-modified probes A and B were purified by a magnetic-separation process. Tosyl-modified magnetic beads (diameter 1 $\mu$m, Invitrogen) were functionalized by amine-modified target oligonucleotide sequences complementary to the target-capturing sequences for cores A and B, respectively. Only the core particles having target-capturing sequences bound thereto could be separated by the magnetic beads. Next, purified probe A and B solutions were hybridized with a sufficient amount of the target sequence in 0.3 M PBS.

Likewise, oligonucleotide-modified Au-Ag core-shell nanoparticle dimers labeled with the Raman active molecule FAM or Dabcy1 were prepared.

## EXAMPLE 2: UV-Visible Spectroscopy and HR-TEM Imaging Analysis

The formation of Au nanoparticle dimers (Cy3 used as a Raman Active molecule) was verified by UV-visible spectroscopy and high-resolution transmission electron microscope (HRTEM) images (FIG. 2). The UV-visible spectra show a very small red-shift after dimer formation, which is in agreement with the previously reported results by Oaul Alivisatos, et al. (Angew chem. 1999.38(12), 1808). FIG. 2A is of typical HR-TEM images of the Au nanoparticle dimers. By a statistical analysis of at least 200 particles, it was found that 25% of the particles existed as a monomer and 65% of the particles as a dimer, and less than 10% as a multimer (trimer, tetramer and so on). The interpaticle distance between the gold particles was found to be ca. 2 - 3 nm as measured by HR-TEM. In a solution (0.3M PBS), the interparticle distance was ~15 nm which was expected to be far longer than that under dried conditions. Also, silver nanoparticles were introduced at a nanometer scale to the surface of the Au nanoparticle dimer by a well-known method (Chem. Comm. 2008, J. Phys. Chem. B 2004, 108, 5882-5888) on the Au nanoparticle surface to shorten the distance between the nanoparticles, which leads to the amplification of SERS signals (see Example 1). Au-Ag core-shell monomers with an Ag-shell thickness of ~3 nm and -10 nm (1a and 1b in FIG. 2C) were also synthesized from a purified probe B solution (30 nm AuNP) under a similar condition. UV-visible spectra from individual solutions were separated at a plasmon resonance peak of -400 nm according to shell thickness. FIG. 2C is of HR-TEM images taken from individual Au-Ag core-shell heterodimers with a diameter of 26 nm - 36 nm (FIG. 2C-2), 30 nm - 40 nm (FIG. 2C-3) and 40 nm - 50 nm (FIG. 2C-3) for a set of two core-shell nanoparticle spheres. FIGS. 2C-1a and 1b are of HR-TEM images taken from individual Au-Ag core-shell monomers with a diameter of 40 nm (shell thickness = 5 nm, 2C-1a) and 50 nm (shell thickness = 10nm, 2C-1b).

Next, the monomeric or dimeric core-shell nanostructures (using Cy3 as a Raman active molecule) were measured for SERS/AFM. In a typical experiment, aliquots (20 $\mu$L) of the Au-Ag core-shell heterodimer solutions washed by repeated centrifugation (8,000 rpm, 20 min, three times) were applied to a poly-L-lysine-coated glass substrate by spin coating, washed many times with nanopure water, and dried in air. Immediately after being prepared, the samples were measured for AFM and SERS. SERS spectra were recorded using an AFM-correlated nano-Raman microscope equipped with an inverted optical microscope (Axovert 200, Zeiss) and a piezoelectric x-y sample scanner (Physik Instrument) was manipulated by an independent homemade scanning controller. The 514.5 nm line of an argon ion laser (Melles Griot) was used as the excitation source coupled with a single-mode optical fibre. A dichroic mirror (520DCLP, Chroma Technology Corp.) was set to direct the excitation laser beam from 50 nW to 1mW into the oil-immersion microscope objective (x100, 1.6 NA, Zeiss), which focused the beam to a diffraction-limited spot (250 nm) on the upper surface of the cover glass slip. The AFM (Bioscope, Digital Instruments, Veeco Metrology Group) with a Nanoscope IV controller was mounted on a micromechanical stage. The tapping-mode AFM module on top of the optical microscope stage was used to correlate the Raman signal with the AFM topographical image with an overlap precision of <100 nm. The laser focal spot was exactly matched with the center of the AFM tip for symmetrical scattering on the AFM tip end. The background Raman signals were collected on a liquid-nitrogen-cooled (-125°C) CCD (charge-coupled device). The scattering spectra of the sample were recorded in the range of 500 - 2,000 cm$^{-1}$, in one acquisition, 1 s accumulations and 400 $\mu$W. All of the data were baseline-corrected by subtracting the background signals from Si.

## EXAMPLE 3: AFM (Atomic Force Micrograph) Analysis of Au-Ag Core-Shell Nanoparticles

FIG. 3A shows magnified AFM images (1 X 1 $\mu$m) of the core-shell monomer and heterodimer nanostructures (using Cy3 as a Raman active molecule), which were coincident in shape and diameter with HR-TEM images. FIG. 3B shows the correlated SERS spectra from the corresponding single AFM-imaged particles in FIG. 3A. No Raman signals were detected for the monomeric Au-Ag core-shell nanoparticles with 5 nm (FIG. 3A-1) and 10 nm (FIG. 3A-2) silver shells, respectively because they had no hot spots and only one Cy3 molecule per particle. The Au nanoparticle heterodimers without Ag shells or with a gap distance less than 1 nm therebetween did not show any detectable SERS signal either. This is due to insufficient electromagnetic enhancement under 514.5 nm laser excitation conditions. When the Ag shell thickness was < 3 nm (FIG. 3A-4), no Raman signals were detected even after using an elevated incident laser power (- 200 $\mu$W). These results indicate that a thin silver shell (<3 nm) could not induce sufficient electromagnetic enhancements in SERS.

In contrast, when the Ag shell thickness was -5 nm (FIG. 3A-5), a strong SERS signal from a Cy3 label, located in the junction of two core-shell particles, was observed. The characteristic Raman peaks for Cy3 dye, although low in intensity, were observed at 1,470 and 1,580 $cm^{-1}$, characteristic of fingerprint spectra, from a 514.5 nm laser excitation. The low intensity of these peaks is probably due to the presence of only one molecule within the hot junction region and relatively low laser power (100 $\mu$W) compared with the power intensity used in other single-molecule SERS studies (Science 1997. 275(5203), 1102, Phys rev lett 1997, 78(9), 1667, Nano lett 2006, 6(1) 2173, Nano lett DOI: 10.1021/ni803621x). Signals were taken from different species as in the oligonucleotide-modified Au nanoparticles. FIG. 3C shows the comparison of SERS spectra of Cy3-modified oligonucleotides (5-HS- $(CH_2)_6$-$A_{10}$-$PEC_{18}$-ATCCTTAT-CAATATTAAA-Cy3-3', 1 nM, red line) with those of Cy3-free oligonucleotides (5-HS-$(CH_2)_6$-$A_{10}$-$PEG_{18}$-ATCCTTAT-CAATATTAAA-3', 1 nM, black line) in aggregated Ag colloids. The SERS spectra of FIG. 3C (black line) features predominant adenine peaks (734 $cm^{-1}$, 1320 $cm^{-1}$) over other bases due to the enrichment of the adenine base (A10 used as a spacer sequence) (JACS 2008, 130(16), 5523). It is important that the lowest detection limit for non-adenine DNA bases, reported so far, is in the sub-micromolar range (JACS, 2006, 128, 15580). However, relatively strong signals were read at 1,470 $cm^{-1}$ and 1,580 $cm^{-1}$, characteristic of the Cy3 molecule, as shown in the SERS spectra of FIG. 3C (red line) (Anal chem. 2004, 76, 412-417). It is known that the Raman intensity and spectral positions of Cy3 molecules fluctuate with time, and the Raman spectra are different for each observed nanostructure (J. Phys. Chem. B 2002, 106, 8096). Therefore, spectral patterns are comparable, but not fully compatible with the reported ones. Irrespective of shell thickness, no observations of detectable SERS signals from the Au-Ag core-shell monomers under the experimental condition indicated that only Cy molecules localized at the interparticle junction could induce SERS peaks at 1,470 and 1,580 $cm^{-1}$. As shown in Raman spectra taken from the core-shell dimers with a shell thickness of ~10 nm (FIG. 3A-6), predominant adenine peaks were observed at 734 $cm^{-1}$ and 1320 $cm^{-1}$ along with a Cy3 peak at 1,480 $cm^{-1}$. Raman scattering intensities from other nanoparticles were not observed in a specific form. A thick Ag shell could compensate for Cy3 molecules which caused improper electromagnetic enhancement.

**EXAMPLE 4: Analysis of SERS Spectra from Au-Ag Core-Shell Nanoparticle Dimers According to Polarization of Incident Laser**

Most of the core-shell nanoparticle dimers with a shell thickness of -5 nm (using Cy3 as a Raman active molecule) showed detectable SERS signals from single molecules, as shown in FIGS. 4A and 4B. Considering that the incident laser light is not exactly polarized to the interparticle axes of the dimer (panels 1-5 in FIG. 4A), detectable SERS signals from each of the perpendicularly polarized nanoparticle dimers on the same surface were observed. However, FIG. 5C shows only small peaks at 1,470 $cm^{-1}$ because the dimer orientation is nearly perpendicular to the incident light. Herein, it was found that the core-shell nanoparticle dimers with the shell thickness optimized might be of hot spot structures highly applicable to the detection of a single DNA molecule.

It is experimentally known that on-off blinking behaviors are observed upon single-molecule detection (FIG. 5A) (J. Phys. Chem. B2002, 106, 8096). The absence of Raman intensity continues for 10 sec, after which Raman intensity is in an ON state. This On-Off cycling phenomenon may be repeated for several minutes during which signals ultimately disappear from an intense field. The SERS intensity fluctuation was observed on a second timescale owing to molecular movement around the hot spot. These blinking and fluctuation phenomena were in agreement with previous reports.

FIGS. 5C and 5D show the incident laser polarization dependence of the Raman signals for the Au-Ag core-shell dimer. All of the spectra were taken with a 514.5nm excitation laser, 20 s accumulation time and 40 $\mu$W laser power. Maximum Cy3 peaks were observed when the incident laser light was polarized parallel to the longitudinal axis of the heterodimer. When the laser light was rotated by 20-90° away from the longitudinal axis, the Cy3 signal was gradually reduced. The Raman peaks disappeared when the laser polarized perpendicular to the longitudinal axis (that is, 90° and 270°). The enhancement factor (EF) at 1,580 $cm^{-1}$ of the hot spot in the dimeric nanostructure was calculated according to the following equation.

$$EF = (I_{sers} \times N_{bulk}) / (I_{bulk} \times N_{molecule})$$

wherein

$I_{sers}$ and $I_{bulk}$ represent the same intensity of bands for SERS and bulk spectra, respectively,
$N_{bullk}$ is the number density of bulk molecules in a bulk sample, and
$N_{molecule}$ is the number density of Cy3 in SERS spectra (Nmolecule=1). The strongest spectrum band was read at 1,580 $cm^{-1}$ regions, so that it was used as the intensity for $I_{sers}$ and $I_{bulk}$. In this manner, the EF of the hot spot was calculated to be 2.7 $\times 10^{12}$.

On the other hand, highly sensitive SERS spectra were detected from nanoparticle dimers modified with oligonucleotides labeled with FAM and Dabcyl, as described for Cy3. Thus, the dimeric nanostructures and preparation method in accordance with the present invention are applied to general Raman active molecules.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

**Claims**

1. A dimeric nanostructure, comprising two nanoparticles, with a Raman active molecule localized at a junction therebetween, each nanoparticle consisting of a gold or silver core with oligonucleotides attached to the surface thereof, and a gold or silver shell sheathing the core, the particles being linked to each other by the oligonucleotides.

2. The dimeric nanostructure according to claim 1, wherein in each nanoparticle the oligonucleotides are attached at one terminus to a surface of the core while being partially exposed to the outside of the shell.

3. The dimeric nanostructure according to claim 2, wherein the oligonucleotides attached to the surface of each nanoparticle comprises a protecting oligonucleotide and a target-capturing oligonucleotide, wherein the protecting oligonucleotide is an oligonucleotide which is attached to the surface of a core particle, stabilizing the core particle with the aim of allowing the target-capturing oligonucleotide to adhere properly to the core surface and to protect it and wherein the target-capturing oligonucleotide is an oligonucleotide having a sequence complementary to that of a target oligonucleotide.

4. The dimeric nanostructure according to claim 3, wherein the target-capturing oligonucleotides attached to the surface of the respective nanoparticles are hybridized with a target oligonucleotide.

5. The dimeric nanostructure according to claim 1, wherein the oligonucleotide is attached via a surface-bound functional group selected from the group consisting of thiol group, amine group and alcohol group to the surface.

6. The dimeric nanostructure according to claim 5, wherein the oligonucleotide is offset by a spacer sequence from the surface-bound functional group.

7. The dimeric nanostructure according to claim 1, being selected from a group consisting of:

   i) a dimeric nanostructure comprising two nanoparticles, each consisting of a gold core and a silver shell,
   ii) a dimeric nanostructure comprising two nanoparticles, each consisting of a silver core and a gold shell,
   iii) a dimeric nanostructure comprising two nanoparticles, each consisting of a gold core and a gold shell,
   iv) a dimeric nanostructure comprising two nanoparticles, each consisting of a silver core and a silver shell, and
   v) a dimeric nanostructure comprising two nanoparticles, one consisting of a gold core and a silver shell and the other consisting of a silver core and a gold shell.

8. The dimeric nanostructure according to claim 1, wherein the core ranges in diameter from 5 to 300 nm.

9. The dimeric nanostructure according to claim 8, wherein the core ranges in diameter from 10 to 40 nm.

10. The dimeric nanostructure according to claim 1, wherein the shell ranges in thickness from 1 to 300 nm.

11. The dimeric nanostructure according to claim 10, wherein the shell ranges in thickness from 1 to 20 nm.

12. The dimeric nanostructure according to claim 1, wherein the Raman active molecule is selected from a group consisting of FAM, Dabcyl, TRIT (tetramethyl rhodamine isothiol), NBD (7-nitrobenz-2-1,3-diazole), Texas Red dye, phthalic acid, terephthalic acid, isophthalic acid, cresyl fast violet, cresyl blue violet, brilliant cresyl blue, para-aminobenzoic acid, erythrosine, biotin, digoxigenin, 5-carboxy-4',5'-dichloro-2',7'-dimethoxy, fluorescein, 5-carboxy-2',4',5',7'-tetrachlorofluorescein, 5-carboxyfluorescein, 5-carboxyrhodamine, 6-carboxyrhodamine, 6-carboxytetramethyl aminophthalocyanine, azomethine, cyanine, xanthine, succinylfluorescein, aminoacridine, quantum dots, carbon nanotubes, carbon allotropes, cyanide, thiol, chlorine, bromine, methyl, phosphorus, sulfur, cyanine dyes (Cy3, Cy3.5, Cy5), and rhodamine.

13. The dimeric nanostructure according to claim 1, wherein the Raman active molecule is an organic fluorescent molecule.

14. The dimeric nanostructure according to claim 1, being functionalized at a surface thereof or a surface of the core with a probe molecule capable of recognizing an analyte to be analyzed.

15. The dimeric nanostructure according to claim 14, wherein the analyte to be analyzed is selected from among amino acids, peptides, polypeptides, proteins, glycoproteins, lipoproteins, nucleosides, nucleotides, oligonucleotides, nucleic acids, saccharides, carbohydrates, oligosaccharides, polysaccharides, fatty acids, lipids, hormones, metabolites, cytokines, chemokines, receptors, neurotransmitters, antigens, allergens, antibodies, substrates, co-factors, inhibitors, drugs, pharmaceutical substances, nutrients, prions, toxins, toxic substances, explosive substances, pesticides, chemical weapon agents, biologically noxious agents, radioactive isotopes, vitamins, heterocyclic aromatic compounds, oncogenic agents, mutagenic factors, anesthetics, amphetamine, barbiturate, hallucinogens, wastes, and contaminants.

16. The dimeric nanostructure according to claim 14, wherein the probe molecule is selected from among antibodies, antibody fragments, soluble proteins, ligand proteins, enzymes, inhibitor proteins, cell-adhesion proteins, oligonucleotides, polynucleotides, nucleic acids, and aptamers.

17. The dimeric nanostructure according to claim 1, being entirely coated with an inorganic substance.

18. The dimeric nanostructure according to claim 17, wherein the inorganic substance is silica.

19. A method for constructing the dimeric nanostructure of claim 1, comprising:

1) synthesizing core A and core B, respectively, the core A having a protecting oligonucleotide and a target-capturing oligonucleotide which are bound to a surface thereof, the core B having a protecting oligonucleotide and a target-capturing oligonucleotide modified at one terminus with a Raman active molecule which is bound to a surface thereof, wherein the protecting oligonucleotide is an oligonucleotide which is attached to the surface of a core particle, stabilizing the core particle with the aim of allowing the target-capturing oligonucleotide to adhere properly to the core surface and to protect it and wherein the target-capturing oligonucleotide is an oligonucleotide having a sequence complementary to that of a target oligonucleotide;
2) hybridizing the core A and the core B with a target oligonucleotide to form a dimeric structure; and
3) introducing a shell on each of the core A and the core B.

20. The method according to claim 19, wherein the step 1 further comprises the step of
separating nanoparticles only which the target-capturing oligonucleotide is bound to in the core A and core B by hybridizing with magnetic microparticles having complementary sequence of the target-capturing oligonucleotide of core A and core B.

21. The method according to claim 19, wherein the introduction of the shell is achieved by reacting the core with a shell precursor in the presence of a reducing agent and a stabilizer.

22. Use of the dimeric nanostructure according to any of claims 1 to 18 for detecting an analyte, comprising:

1) functionalizing a surface of the dimeric nanostructure or the core of the dimeric nanostructure with a probe molecule capable of detecting an analyte;
2) exposing the dimeric nanostructure to a sample containing at least one analyte; and
3) detecting and identifying the analyte by laser excitation and Raman spectroscopy.

**Patentansprüche**

1. Dimere Nanostruktur, umfassend zwei Nanopartikel, wobei ein Ramanaktives Molekül an einer Verbindung zwischen ihnen lokalisiert ist, wobei jeder Nanopartikel aus einem Gold- oder Silberkern, an dessen Oberfläche Oligonukleotide befestigt sind, und einer Gold- oder Silberhülle besteht, die den Kern ummantelt, wobei die Partikel durch die Oligonukleotide miteinander verbunden sind.

2. Dimere Nanostruktur nach Anspruch 1, wobei in jedem Nanopartikel die Oligonukleotide mit einem Terminus an einer Oberfläche des Kerns befestigt sind, während sie teilweise auf der Außenseite der Hülle exponiert sind.

3. Dimere Nanostruktur nach Anspruch 2, wobei die Oligonukleotide, die an die Oberfläche von jedem Nanopartikel befestigt sind, ein schützendes Oligonukleotid und ein zieleinfangendes Oligonukleotid umfassen, wobei das schützende Oligonukleotid ein Oligonukleotid ist, das an der Oberfläche von einem Kernpartikel befestigt ist, wobei es den Kernpartikel mit dem Ziel stabilisiert dem zieleinfangenden Oligonukleotid eine korrekte Adhäsion an die Kernoberfläche zu erlauben und es zu schützen, und wobei das zieleinfangende Oligonukleotid ein Oligonukleotid ist, das eine Sequenz hat, die zu der Sequenz von einem Zieloligonukleotid komplementär ist.

4. Dimere Nanostruktur nach Anspruch 3, wobei die zieleinfangenden Oligonukleotide, die an die Oberfläche der entsprechenden Nanopartikel befestigt sind, mit einem Zieloligonukleotid hybridisiert sind.

5. Dimere Nanostruktur nach Anspruch 1, wobei das Oligonukleotid an die Oberfläche über eine oberflächengebundene funktionale Gruppe befestigt ist, die ausgewählt aus der Gruppe bestehend aus einer Thiolgruppe, Amingruppe und Alkoholgruppe.

6. Dimere Nanostruktur nach Anspruch 5, wobei das Oligonukleotid von der oberflächengebundenen funktionalen Gruppe durch einen Distanzhalt abgesetzt ist.

7. Dimere Nanostruktur nach Anspruch 1, welche ausgewählt ist aus einer Gruppe bestehend aus einer:

   i) dimeren Nanostruktur, umfassend zwei Nanopartikel, wobei jeder davon aus einem Goldkern und einer Silberhülle besteht,
   ii) dimeren Nanostruktur, umfassend zwei Nanopartikel, wobei jeder davon aus einem Silberkern und einer Goldhülle besteht,
   iii) dimeren Nanostruktur, umfassend zwei Nanopartikel, wobei jeder davon aus einem Goldkern und einer Goldhülle besteht,
   iv) dimeren Nanostruktur, umfassend zwei Nanopartikel, wobei jeder davon aus einem Silberkern und einer Silberhülle besteht, und
   v) dimeren Nanostruktur, umfassend zwei Nanopartikel, wobei einer davon aus einem Goldkern und einer Silberhülle besteht und der andere aus einem Silberkern und einer Goldhülle besteht.

8. Dimere Struktur nach Anspruch 1, wobei der Durchmesser des Kerns von 5 bis 300 nm reicht.

9. Dimere Nanostruktur nach Anspruch 8, wobei der Durchmesser des Kerns von 10 bis 40 nm reicht.

10. Dimere Nanostruktur nach Anspruch 1, wobei die Dicke der Hülle von 1 bis 300 nm reicht.

11. Dimere Nanostruktur nach Anspruch 10, wobei die Dicke der Hülle von 1 bis 20 nm reicht.

12. Dimere Nanostruktur nach Anspruch 1, wobei das Raman-aktive Molekül ausgewählt ist aus der Gruppe, bestehend aus FAM, Dabcyl, TRIT (Tetramethylrhodaminisothiol), NBD (7-Nitrobenz-2-1,3-diazol), Texas-Red-Farbstoff, Phthalsäure, Terephthalsäure, Isophthalsäure, Kresylechtviolett, Kresylblauviolett, Brilliantkresylblau, para-Aminobenzoesäure, Erythrosin, Biotin, Digoxigenin, 5-Carboxy-4',5'-dichlor-2',7'-dimethoxyfluoreszein, 5-Carboxy-2',4',5',7'-tetrachlorfluoreszein, 5-Carboxyfluoreszein, 5-carboxyrhodamin, 6-Carboxyrhodamin, 6-Carboxytetramethylamino-phthalocyanin, Azomethin, Cyanin, Xanthin, Succinylfluoreszein, Aminoacridin, Quantenpunkten, Kohlenstoffnanoröhrchen, Kohlenstoffallotrope, Zyanid, Thiol, Chlor, Brom, Methyl, Phosphor, Schwefel, Cyaninfarbstoff (Cy3, Cy3.5, Cy5) und Rhodamin.

13. Dimere Nanostruktur nach Anspruch 1, wobei das Raman-aktive Molekül ein organisches fluoreszierendes Molekül ist.

14. Dimere Nanostruktur nach Anspruch 1, die an einer Oberfläche von ihr oder an einer Oberfläche von dem Kern mit einem Sondenmolekül funktionalisiert ist, das in der Lage ist einen Analyten, der analysiert werden soll, zu erkennen.

15. Dimere Nanostruktur nach Anspruch 14, wobei der Analyt, der analysiert werden soll, ausgewählt ist aus Aminosäuren, Peptiden, Polypeptiden, Proteinen, Glykoproteinen, Lipoproteinen, Nukleosiden, Nukleotiden, Oligonukleotiden, Nukleinsäuren, Sacchariden, Carbohydraten, Oligosacchariden, Polysacchariden, Fettsäuren, Lipiden, Hormonen, Metaboliten, Zytokinen, Chemokinen, Rezeptoren, Neurotransmittern, Antigenen, Allergenen, Antikörpern, Substraten, Cofaktoren, Inhibitoren, Medikamenten, pharmazeutischen Substanzen, Nährstoffen, Prionen, Toxinen, toxischen Substanzen, explosiven Substanzen, Pestiziden, Mitteln für chemische Waffen, biologisch schädlichen Mitteln, radioaktiven Isotopen, Vitaminen, heterozyklischen aromatischen Verbindungen, onkogenen Mitteln, mutagenen Faktoren, Anästhetika, Amphetamin, Barbiturat, Halluzinogenen, Abfällen und Verunreinigungen.

16. Dimere Nanostruktur nach Anspruch 14, wobei das Sondenmolekül ausgewählt ist aus Antikörpern, Antikörperfragmenten, löslichen Proteinen, Ligandenproteinen, Enzymen, Inhibitorproteinen, Zelladhäsionsproteinen, Oligonukleotiden, Polynukleotiden, Nukleinsäuren und Aptameren.

17. Dimere Nanostruktur nach Anspruch 1, die vollständig mit einer anorganischen Substanz beschichtet ist.

18. Dimere Nanostruktur nach Anspruch 17, wobei die anorganische Substanz Siliziumdioxid ist.

19. Verfahren zum Herstellen der dimeren Nanostruktur nach Anspruch 1, umfassend:

1) Synthetisieren des Kerns A beziehungsweise Kerns B, wobei der Kern A ein schützendes Oligonukleotid und ein zieleinfangendes Oligonukleotid hat, die auf einer Oberfläche des Kerns A gebunden sind, und der Kern B ein schützendes Oligonukleotid und ein zieleinfangendes Oligonukleotid hat, das an einem Terminus mit einem Raman-aktiven Molekül modifiziert ist und das an eine Oberfläche des Kerns B gebunden ist, wobei das schützende Oligonukleotid ein Oligonukleotid ist, das an der Oberfläche von einem Kernpartikel befestigt ist, wobei es den Kernpartikel mit dem Ziel stabilisiert, dem zieleinfangenden Oligonukleotid eine korrekte Adhäsion an die Kernoberfläche zu erlauben und es zu schützen, und wobei das zieleinfangende Oligonukleotid ein Oligonukleotid ist, das eine Sequenz hat, die zu der Sequenz von einem Zieloligonukleotid komplementär ist;
2) Hybridisieren des Kerns A und des Kerns B mit einem Zieloligonukleotid, um eine dimere Struktur zu bilden; und
3) Einführen einer Hülle auf jeweils den Kern A und den Kern B.

20. Verfahren nach Anspruch 19, wobei der Schritt 1 ferner den Schritt umfasst
Trennen von ausschließlich Nanopartikeln, an die das zieleinfangende Oligonukleotid in dem Kern A und dem Kern B gebunden ist, mittels Hybridisieren mit magnetischen Mikropartikeln, die eine komplementäre Sequenz zu dem zieleinfangenden Oligonukleotid des Kerns A und des Kerns B haben.

21. Verfahren nach Anspruch 19, wobei das Einführen der Hülle erreicht wird, indem der Kern mit einem Hüllenvorläufer in der Anwesenheit von einem Reduktionsmittel und einem Stabilisator reagiert wird.

22. Verwendung der dimeren Nanostruktur nach einem der Ansprüche 1 bis 18 zum Nachweisen eines Analyten, umfassend:

1) Funktionalisieren einer Oberfläche von der dimeren Nanostruktur oder des Kerns von der dimeren Nanostruktur mit einem Sondenmolekül, das in der Lage ist, den Analyten detektieren;
2) Exponieren der dimeren Nanostruktur zu einer Probe, die mindestens einen Analyten enthält; und
3) Nachweisen und Identifizieren des Analyten mittels Laseranregung und Raman-Spektroskopie.

**Revendications**

1. Nanostructure dimérique, comprenant deux nanoparticules, avec une molécule à activité Raman localisée à une jonction entre celles-ci, chaque nanoparticule étant constituée d'un noyau d'or ou d'argent avec des oligonucléotides liés à la surface de celui-ci, et une enveloppe d'or ou d'argent recouvrant le noyau, les particules étant mutuellement liées par les oligonucléotides.

2. Nanostructure dimérique selon la revendication 1, dans laquelle, dans chaque nanoparticule, les oligonucléotides sont liés à une extrémité à une surface du noyau tout en étant partiellement exposés à l'extérieur de l'enveloppe.

3. Nanostructure dimérique selon la revendication 2, dans laquelle les oligonucléotides liés à la surface de chaque nanoparticule comprennent un oligonucléotide protecteur et un oligonucléotide de capture de cible, l'oligonucléotide protecteur étant un oligonucléotide qui est lié à la surface d'une particule de noyau, stabilisant la particule de noyau dans le but de permettre à l'oligonucléotide de capture de cible d'adhérer correctement à la surface du noyau et de le protéger, et l'oligonucléotide de capture de cible étant un oligonucléotide ayant une séquence complémentaire de celle d'un oligonucléotide cible.

4. Nanostructure dimérique selon la revendication 3, dans laquelle les oligonucléotides de captures de cible liés à la surface des nanoparticules respectives sont hybridés avec un oligonucléotide cible.

5. Nanostructure dimérique selon la revendication 1, dans laquelle l'oligonucléotide est lié par l'intermédiaire d'un groupe fonctionnel lié à la surface choisi dans le groupe constitué d'un groupe thiol, un groupe amine et un groupe alcool à la surface.

6. Nanostructure dimérique selon la revendication 5, dans laquelle l'oligonucléotide est décalé par une séquence d'espaceur par rapport au groupe fonctionnel lié à la surface.

7. Nanostructure dimérique selon la revendication 1, étant choisie dans un groupe constitué de :

i) une nanostructure dimérique comprenant deux nanoparticules, chacune étant constituée d'un noyau d'or et d'une enveloppe d'argent,
ii) une nanostructure dimérique comprenant deux nanoparticules, chacune étant constituée d'un noyau d'argent et d'une enveloppe d'or,
iii) une nanostructure dimérique comprenant deux nanoparticules, chacune étant constituée d'un noyau d'or et d'une enveloppe d'or,
iv) une nanostructure dimérique comprenant deux nanoparticules, chacune étant constituée d'un noyau d'argent et d'une enveloppe d'argent, et
v) une nanostructure dimérique comprenant deux nanoparticules, l'une étant constituée d'un noyau d'or et d'une enveloppe d'argent et l'autre étant constituée d'un noyau d'argent et d'une enveloppe d'or.

8. Nanostructure dimérique selon la revendication 1, dans laquelle le noyau a un diamètre dans la plage de 5 à 300 nm.

9. Nanostructure dimérique selon la revendication 8, dans laquelle le noyau a un diamètre dans la plage de 10 à 40 nm.

10. Nanostructure dimérique selon la revendication 1, dans laquelle l'enveloppe a une épaisseur dans la plage de 1 à 300 nm.

11. Nanostructure dimérique selon la revendication 10, dans laquelle l'enveloppe a une épaisseur dans la plage de 1 à 20 nm.

12. Nanostructure dimérique selon la revendication 1, dans laquelle la molécule à activité Raman est choisie dans un groupe constitué de FAM, Dabcyl, TRIT (tétraméthyl-rhodamine-isothiol), NBD (7-nitrobenz-2-1,3-diazole), colorant rouge Texas, acide phtalique, acide téréphtalique, acide isophtalique, violet de crésyl rapide, bleu-violet de crésyl, bleu de crésyl brillant, acide para-aminobenzoïque, érythrosine, biotine, digoxigénine, 5-carboxy-4',5'-dichloro-2',7'-diméthoxyfluorescéine, 5-carboxy-2',4',5',7'-tétrachlorofluorescéine, 5-carboxyfluorescéine, 5-carboxyrhodamine, 6-carboxyrhodamine, 6-carboxytétraméthyl-aminophtalocyanine, azométhine, cyanine, xanthine, succinylfluorescéine, aminoacridine, points quantiques, nanotubes de carbone, allotropes de carbone, cyanure, thiol, chlore, brome, méthyle, phosphore, soufre, colorants de cyanine (Cy3, Cy3.5, Cy5), et rhodamine.

13. Nanostructure dimérique selon la revendication 1, dans laquelle la molécule à activité Raman est une molécule fluorescente organique.

14. Nanostructure dimérique selon la revendication 1, étant fonctionnalisée à une surface de celle-ci ou une surface du noyau avec une molécule de sonde capable de reconnaître un analyte à analyser.

15. Nanostructure dimérique selon la revendication 14, dans laquelle l'analyte à analyser est choisi parmi des acides aminés, des peptides, des polypeptides, des protéines, des glycoprotéines, les lipoprotéines, des nucléosides, des nucléotides, des oligonucléotides, des acides nucléiques, des saccharides, des glucides, des oligosaccharides, des polysaccharides, des acides gras, des lipides, des hormones, des métabolites, des cytokines, des chimiokines, des récepteurs, des neurotransmetteurs, des antigènes, des allergènes, des anticorps, des substrats, des cofacteurs, des inhibiteurs, des médicaments, des substances pharmaceutiques, des nutriments, des prions, des toxines, des substances toxiques, des substances explosives, des pesticides, des agents d'armes chimiques, des agents biologiquement nocifs, des isotopes radioactifs, des vitamines, des composés aromatiques hétérocycliques, des agents oncogènes, des facteurs mutagènes, des anesthésiques, une amphétamine, un barbiturique, des hallucinogènes, des déchets et des contaminants.

16. Nanostructure dimérique selon la revendication 14, dans laquelle la molécule de sonde est choisie parmi des anticorps, des fragments d'anticorps, des protéines solubles, des protéines de ligand, des enzymes, des protéines inhibitrices, des protéines d'adhésion cellulaire, des oligonucléotides, des polynucléotides, des acides nucléiques et des aptamères.

17. Nanostructure dimérique selon la revendication 1, étant entièrement revêtue d'une substance inorganique.

18. Nanostructure dimérique selon la revendication 17, dans laquelle la substance inorganique est la silice.

19. Procédé de construction de la nanostructure dimérique de la revendication 1, comprenant :

   1) la synthèse d'un noyau A et d'un noyau B, respectivement, le noyau A ayant un oligonucléotide protecteur et un oligonucléotide de capture de cibles qui sont liées à une surface de celui-ci, le noyau B ayant un oligonucléotide protecteur et un oligonucléotide de capture de cibles modifiées à une extrémité avec une molécule à activité Raman qui est liée à une surface de celui-ci, l'oligonucléotide protecteur étant un oligonucléotide qui est lié à la surface d'une particule de noyau, stabilisant la particule de noyau dans le but de permettre à l'oligonucléotide de capture de cible d'adhérer correctement à la surface du noyau et de le protéger, et l'oligonucléotide de capture de cible étant un oligonucléotide ayant une séquence complémentaire de celle d'un oligonucléotide cible ;
   2) l'hybridation du noyau A et du noyau B avec un oligonucléotide cible pour former une structure dimérique ; et
   3) l'introduction d'une enveloppe sur chacun du noyau A et du noyau B.

20. Procédé selon la revendication 19, dans lequel l'étape 1 comprend en outre l'étape de séparation uniquement des nanoparticules auxquelles l'oligonucléotide de capture de cible est lié dans le noyau A et le noyau B par hybridation avec des microparticules magnétiques ayant une séquence complémentaire de l'oligonucléotide de capture de cible du noyau A et du noyau B.

21. Procédé selon la revendication 19, dans lequel l'introduction de l'enveloppe est effectuée par réaction du noyau avec un précurseur d'enveloppe en présence d'un agent réducteur et d'un stabilisant.

22. Utilisation de la nanostructure dimérique selon l'une quelconque des revendications 1 à 18 pour détecter un analyte, comprenant :

   1) la fonctionnalisation d'une surface de la nanostructure dimérique ou du noyau de la nanostructure dimérique avec une molécule de sonde capable de détecter un analyte ;
   2) l'exposition de la nanostructure dimérique à un échantillon contenant au moins un analyte ; et
   3) la détection et l'identification de l'analyte par excitation laser et spectroscopie Raman.

[FIG. 1]

[FIG. 2]

[FIG. 3]

[FIG. 4]

[FIG. 5]

[FIG. 6]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03008539 A
- WO 2009136741 A
- US 6002471 A
- US 6040191 A
- US 6149868 A
- US 6174677 B
- US 6313914 B
- US 5306403 A

### Non-patent literature cited in the description

- *Science,* 1997, vol. 275 (5303), 1102
- *Phys rev lett,* 1997, vol. 78 (9), 1667
- *science,* 2002, vol. 297, 1536
- *J Phys Chem B,* 2002, vol. 106 (2), 311
- *Science,* 2008, vol. 321, 388
- **KAHRAMAN et al.** *Plasmonics,* 2009, vol. 4, 293
- **YAN CUI et al.** *J. Phys. Chem B,* 2006, vol. 110, 4002
- **HUO et al.** *Anal. Chem.,* 2007, vol. 79, 916
- **FELDHEIM.** *The Electrochemical Society Interface, Fall,* 2011, 22-25
- *Chem. Comm.,* 2008
- *J. Phys. Chem. B,* 2004, vol. 108, 5882-5888
- **OAUL ALIVISATOS et al.** *Angew chem.,* 1999, vol. 38 (12), 1808
- *Science,* 1997, vol. 275 (5203), 1102
- *Nano lett,* 2006, vol. 6 (1), 2173
- *JACS,* 2008, vol. 130 (16), 5523
- *JACS,* 2006, vol. 128, 15580
- *Anal chem.,* 2004, vol. 76, 412-417
- *J. Phys. Chem. B,* 2002, vol. 106, 8096